(19) Europäisches Patentamt
European Patent Office
Office européen des brevets

(11) **EP 2 143 385 A1**

(12) **EUROPÄISCHE PATENTANMELDUNG**

(43) Veröffentlichungstag:
**13.01.2010 Patentblatt 2010/02**

(21) Anmeldenummer: **09151323.4**

(22) Anmeldetag: **26.01.2009**

(51) Int Cl.:
***A61B 8/08*** *(2006.01)*

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(30) Priorität: **09.07.2008 DE 102008040266**

(71) Anmelder: **Biotronik CRM Patent AG**
**6341 Baar (CH)**

(72) Erfinder:
- **Skerl, Olaf**
  **18209, Bad Doberan (DE)**
- **Lippert, Michael**
  **91522, Ansbach (DE)**
- **Czygan, Gerald**
  **91054, Buckenhof (DE)**

(74) Vertreter: **Lindner-Vogt, Karin L.**
**Biotronik SE & Co. KG**
**Woermannkehre 1**
**12359 Berlin (DE)**

(54) **Implantierbare Messanordnung**

(57)    Implantierbare Messanordnung zur intrakorporalen akustischen Messung von geometrischen Parametern und Bewegungsparametern in und an Organen und/ oder Geweben eines Patienten, aufweisend ein implantierbares Gerät, insbesondere ein elektromedizinisches Gerät; einen implantierbaren Schallwandler zum Abstrahlen und Empfangen von Ultraschallwellen, der in Signalverbindung mit dem implantierbaren Gerät steht; und einen mit dem implantierbaren Gerät verbundenen und vom Schallwandler entfernt angeordneten implantierbaren Reflektor zum Zurückstrahlen die Ultraschallwellen in die Richtung des Schallwandlers, wobei das elektromedizinische Gerät Mittel zur Auswertung der vom Schallwandler aufgenommenen zurückgestrahlten Ultraschallwellen aufweist.

Fig. 2

EP 2 143 385 A1

**Beschreibung**

Die Erfindung betriff eine implantierbare Messanordnung zur intrakorporalen akustischen Messung von geometrischen Parametern und/oder Bewegungsparametern in und an Organen/Geweben eines Patienten. Sie betrifft des Weiteren eine Anordnung zur Fernüberwachung von physiologischen Messgrößen eines Patienten, die auf einer derartigen Messanordnung aufbaut.

Ultraschall-Verfahren gehören heute zu den Standardverfahren der medizinischen Diagnostik. Zur Untersuchung der unterschiedlichsten Organe werden dabei am häufigsten bildgebende Verfahren verwendet. Mit den bildgebenden Verfahren können geometrische Parameter bestimmt werden, wie die Größe oder die relative Lage zu anderen Organen. Anhand der Echoamplitude in bestimmten Bereichen können darüber hinaus Informationen über die Gewebestruktur in diesen Bereichen gewonnen werden.

Ultraschall-Techniken auch speziell in der Kardiologie gehören heute zu den wichtigsten Diagnoseverfahren. Dabei werden Laufzeitverfahren und bildgebende Verfahren (Echokardiografie) genutzt, um geometrische Parameter des Herzens zu bestimmen. Informationen über den Blutfluss liefern Doppler-Verfahren (Doppler-Sonografie) und die Bewegungen der Herzwände werden mit dem sog. Tissue-Doppler untersucht. Bei der Echokardiographie können damit beispielsweise die Dimensionen des linken Ventrikels (LV) während der Systole und während der Diastole bestimmt werden, woraus sich u. a. das Schlagvolumen (SV) ermitteln lässt.

Bei Doppler-Verfahren wird über die Frequenzverschiebung der Echos (Doppler-Effekt) die Geschwindigkeit der echogebenden Strukturen bestimmt. Damit kann zum einen die Strömungsgeschwindigkeit des Blutes in einem bestimmten Bereich gemessen werden (Flow-Doppler). Zum anderen kann damit aber auch die Bewegungsgeschwindigkeit von Organen oder Organteilen (z. B. die Bewegung der Herzwände) berührungslos gemessen werden (Tissue-Doppler).

Auch die beiden Doppler-Verfahren stellen heutzutage Standarddiagnoseverfahren dar. Beispielsweise können aus dem mit Hilfe des Flow-Dopplers bestimmten Zeitverlauf der Blutflussgeschwindigkeit im LV Ausflusstrakt Parameter zur Beurteilung der kardialen Hämodynamik gewonnen werden. Mit dem Tissue-Doppler werden dagegen beispielsweise Parameter zur Diagnose des Kontraktionsverhaltens und der Kontraktionsdynamik des Herzens bestimmt. Die mittels der Ultraschallverfahren bestimmten Parameter gelten heute als allgemein klinisch akzeptiert oder werden häufig sogar als Referenzparameter verwendet. Vielfach werden diese Methoden kombiniert verwendet. In den letzten Jahren erlangten intrakardiale Ultraschallmessungen immer mehr Bedeutung, da mit ihnen viel direktere Untersuchungen am Herzen ermöglicht werden. Das legt die Bestrebung nahe, diese Ultraschallmessungen auch in einem implantierbaren medizinischen Gerät (IMD), beispielsweise in einem Herzschrittmacher oder ICD für diagnostische Zwecke oder zur Optimierung der Therapie zu nutzen.

Alle Ultraschallverfahren nutzen Ultraschallwellen, die von Inhomogenitäten der akustischen Impedanz in den Geweben reflektiert oder zurückgestreut werden. Der Reflexionskoeffizient (R), d.h. das Verhältnis der reflektierten zur eingestrahlten Schallenergie, ist umso größer, je größer die Differenz der jeweiligen akustischen Impedanzen $Z_{a1}$ und $Z_{a2}$ ist:

$$R = \frac{(Z_{a1} - Z_{a2})^2}{(Z_{a1} + Z_{a2})^2}.$$

Da die Unterschiede in den akustischen Impedanzen zwischen den verschiedenen Körpergeweben allgemein gering sind, wird nur ein geringer Bruchteil der eingestrahlten Energie als Nutzsignal (Echo) wieder empfangen. Die Detektion des Empfangssignals und dessen Verarbeitung erfordert deshalb einen hohen technischen Aufwand, und die Klassifizierung der Echos ist in der Regel nur durch die Einbeziehung eines erfahrenen Bedieners (in der Regel eines Arztes) zu realisieren.

Nach dem Aussenden des Ultraschallimpulses werden vom Empfänger eine Vielzahl von Echos aus unterschiedlichen Entfernungen und unterschiedlichen Winkeln mit stark variierender Intensität empfangen. Das Empfangssignal enthält demzufolge die Überlagerung aller dieser Echos. Mit Hilfe technischer Mittel kann gegebenenfalls der Entfernungs- und Winkelbereich, aus dem die empfangenen Echos berücksichtigt werden, eingeschränkt werden. Bei den üblichen Ultraschall-Diagnoseverfahren wird der Schallwandler von einem erfahrenen Bediener manuell positioniert und auf die zu untersuchende Region ausgerichtet. Auch die Auswahl und Markierung der Messpositionen erfolgt manuell aufgrund der Beurteilung der Ergebnisse (beispielsweise der Ultraschallbilder) durch den fachkundigen Bediener. Aufwendige technische Maßnahmen können heutzutage den Bediener bei diesen Tätigkeiten unterstützen, die automatische Auswahl der für die Messposition relevanten Echos ist im Allgemeinen nicht möglich.

Bei einigen Anwendungen werden auch kommerziell verfügbare Ultraschall-Kontrastmittel eingesetzt, um die Qualität der Empfangssignale zu verbessern. Ultraschall-Kontrastmittel enthalten beispielsweise kleine Gasblasen, die gegenüber der Umgebung eine hohe Differenz in der akustischen Impedanz aufweisen und somit einen großen Anteil der Schallenergie zurückstreuen. Die damit markierten Gewebe oder Organe lassen sich dadurch deutlich von der Umgebung abgrenzen. Damit lassen sich beispielsweise im Ultraschallbild die ohne Kontrastmittel nicht sichtbaren Blutgefässe darstellen.

Seit einiger Zeit werden auch Anstrengungen unternommen, Schrittmacherelektroden und Katheter zusätzlich zu den bereits üblichen Röntgen-Markierungen mit Ultraschall-Markierungen auszurüsten. Durch diese Markierungen wird die zuverlässige Sichtbarkeit der Elektrode bzw. des Katheters oder deren markierter Bereich in Ultraschallbildern gewährleistet. Damit können beispielsweise bei der Implantation oder der Nachsorge bildgebende Ultraschallverfahren als Alternative zu Röntgenaufnahmen verwendet und dadurch die Strahlenbelastung des Patienten verringert werden.

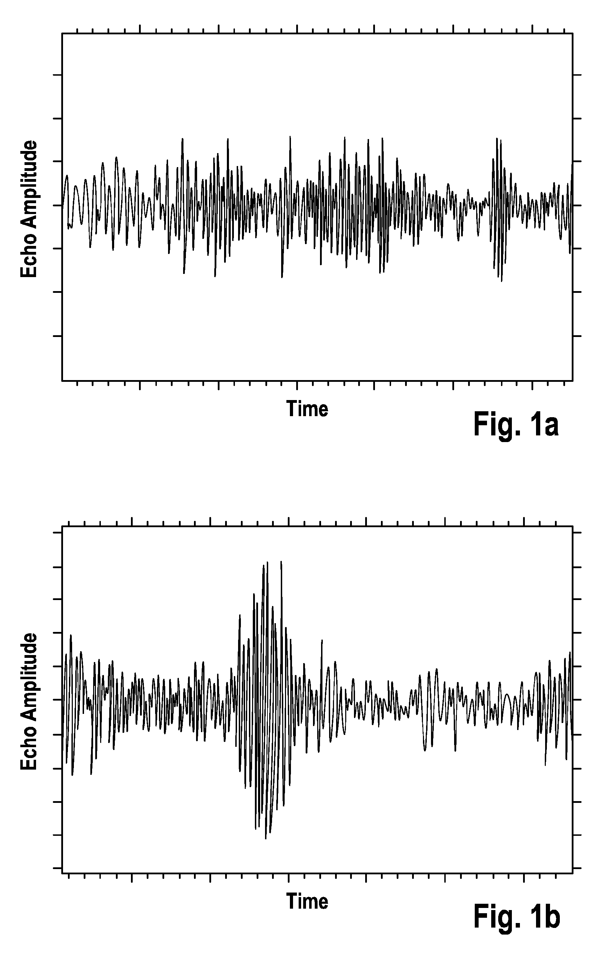

Generell können die Ultraschall-Diagnoseverfahren sowohl nichtinvasiv durch das Auflegen eines Schallwandlers von außen auf den Körper (z.B. transthorakal) als auch invasiv durch das Einführen eines Ultraschallkatheters z.B. in die Blutgefäße (IVUS, Intravascular Ultrasound) durchgeführt werden. Ein Problem bei allen Ultraschalldiagnoseverfahren ist, dass die von den Gewebestrukturen rückgestreute Energie sehr gering ist. Dadurch sind die Echos im Allgemeinen unscharf und von Störsignalen und Rauschen überlagert (s. Fig. 1A). Um die einzelnen Echos zu detektieren und voneinander zu trennen, ist ein hoher Signalverarbeitungsaufwand notwendig.

Die exakte Ausrichtung des Schallstrahls auf die zu untersuchende Region ist ebenfalls von großer Bedeutung. Allgemein wird ein möglichst schmaler Schallstrahl angestrebt. Damit wird gewährleistet, dass das Empfangssignal überwiegend Informationen aus der gewünschten Region enthält und die Zuordnung der Echos zu bestimmten Strukturen möglich wird. Die exakte Positionierung und Ausrichtung der Schallwandler erweist sich bei Implantaten aber als sehr aufwändig. Auch die Langzeitstabilität dieser Positionierung ist nur sehr schwierig sicherzustellen.

Bekannte Verfahren zur Verwendung von Ultraschall in IMD, wie etwa beschrieben in US 5,544,656 oder US 6,421,565, weisen Nachteile auf. Wenn sich hierbei beispielsweise die Ausrichtung der Schallwandler verändert, ist nicht mehr sichergestellt, dass die empfangenen Echos der gewünschten Region entstammen. Anhand der Signale ist diese Veränderung nur unter bestimmten Bedingungen mit großem technischen Aufwand feststellbar.

Um diese Probleme zu lösen, sind Anordnungen bekannt, die mindestens zwei getrennte Schallwandler verwenden, wobei ein Schallwandler als Sender und der zweite (und alle weiteren) Schallwandler als Empfänger arbeitet, vgl. etwa US 6,795,732 oder US 2005/027 323. Diese Anordnung wird häufig als Microsonometer bezeichnet. Die Schallwandler werden dabei so positioniert, dass die gewünschte Diagnoseaufgabe erfüllt wird. Der Vorteil hierbei ist, dass die Empfangswandler den direkt eintreffenden Schall empfangen. Dieses Signal kann mit einfachen Mitteln deutlich von den Störsignalen getrennt werden. Da der Schall jeweils nur von einem Schallwandler gesendet und direkt empfangen wird, ist die Zuordnung der Empfangssignale zu ihrer Quelle und dem Ausbreitungspfad der Schallwelle eindeutig.

Damit ist es möglich, Schallwandler mit sehr breitem Abstrahl- bzw. Empfangswinkel zu verwenden, wodurch an die exakte Ausrichtung und deren Langzeitstabilität nur noch geringe Anforderungen gestellt werden müssen. Der Nachteil dieser Anordnung ist, dass mindestens zwei Schallwandler mit ihren Zuleitungen notwendig sind. Besonders die Zuleitungen stellen bei Langzeitimplantaten oft ein Problem dar und beschränken die Implantationsorte.

Des weiteren werden neuerdings Elektroden und Katheter auch mit zusätzlichen Ultraschallmarkierungen versehen, um ihre Sichtbarkeit in Ultraschallbildern zu verbessern , vgl. dazu US 4,805,628; US 5,383,466, US 5,921,933; US 6,506,156 oder US 7,014,610. Dabei werden beispielsweise in Bereichen der Umhüllung kleine Gasblasen eingeschlossen. Ebenso sind implantierbare Stents mit Ultraschallmarkierungen bekannt (US 5,289,831 oder WO2004/103207). Durch den großen Unterschied in der akustischen Impedanz der Gasblasen im Vergleich zur Umgebung tritt an ihnen eine starke Reflexion der Schallwellen auf. Dadurch liefern diese Ultraschall-Markierungen starke Echos, und sind damit auch für andere intrakorporale Ultraschallmessungen als Reflexionstarget geeignet.

Um Ultraschalldiagnoseverfahren in Langzeitimplantaten mit ihren begrenzten Ressourcen an Energie und Signalverarbeitungsleistung anwenden zu können, sind leicht detektierbare, scharfe Echos von Strukturen an exakt bekannten und stabilen Positionen notwendig.

Der Erfindung liegt daher die Aufgabe der Bereitstellung einer entsprechenden Messanordnung zugrunde, welche überdies im klinischen Einsatz leicht handhabbar und kostengünstig sein soll.

Diese Aufgabe wird durch eine Messanordnung mit den Merkmalen des Anspruchs 1 gelöst. Zweckmäßige Fortbildungen des Erfindungsgedankens sind Gegenstand der abhängigen Ansprüche.

Die Erfindung beinhaltet den Gedanken einer intrakorporalen akustischen Messung von geometrischen und Bewegungsparametern in und an Organen mit Hilfe von definiert platzierten Ultraschall-Reflektoren. Dadurch werden an die Positionierung und Ausrichtung der Ultraschallwandler geringere Anforderungen gestellt als bei bekannten Anordnungen. Es können Schallwandler mit breiter oder kugelförmiger Richtcharakteristik verwendet werden, so dass sich Änderungen der Ausrichtung nur gering auf die Signalqualität auswirken. Der Aufwand für die Signalverarbeitung wird im Vergleich zu bekannten Anordnungen so weit verringert, dass die Empfangssignale mit den begrenzten Ressourcen eines Langzeitimplantats ausgewertet werden können. Ein weiterer Vorteil der erfindungsgemäßen Lösung besteht darin, dass, im Gegensatz zu bekannten Anordnungen, maximal nur ein Schallwandler mit der zugehörigen Zuleitung notwendig ist.

Als Ultraschallreflektoren werden implantierte Körper aus Ultraschallreflektierenden Material, die an definierten Punkten des Körpergewebes fixiert angebracht sind, benutzt. Ebenso können auch beispielsweise Elektroden oder Stents mit Ultraschallmarkierung verwendet werden. Diese Reflektoren liefern signifikante Echos, die mit geringem Aufwand von den Störsignalen getrennt werden können (s. Fig. 1B). Darüber hinaus ist die Position dieser Reflektoren bekannt oder mit allgemein üblichen Methoden ermittelbar.

Die markanten Echos können beispielsweise über eine einfache Amplitudenbewertung aus dem Empfangssignal extrahiert werden. Zusätzlich ist es auch möglich, den Signalbereich für die Detektion des Echos durch ein Zeitfenster einzugrenzen. Diese Anordnung ist nicht auf Ultraschall-Impulsverfahren beschränkt, sie kann auch für kontinuierliche Verfahren (CW Ultraschall) verwendet werden, wobei in diesem Fall der Schallwandler einen Sende- und einen Empfangsteil enthält.

Bei Impulsverfahren kann der Schallwandler auch vom Sende- in den Empfangsbetrieb umgeschaltet werden. Mit dieser Anordnung können sowohl die Schall-Laufzeit vom Sendezeitpunkt bis zum Eintreffen des Echos, die Signalamplitude des Echos als auch die Frequenzverschiebung (Dopplerfrequenz) des Echos oder gleichzeitig alle diese Parameter mit allgemein bekannten Verfahren bestimmt werden. Aus der Schall-Laufzeit lässt sich die jeweilige Entfernung zwischen dem Schallwandler und dem Reflektor ermitteln und damit beispielsweise geometrische Veränderungen des zu beobachtenden Organabschnitts diagnostizieren. Bei konstantem Abstand zwischen Schallwandler und Reflektor lässt sich aus der Schall-Laufzeit die Schallgeschwindigkeit im beobachteten Abschnitt bestimmen, woraus beispielsweise Aussagen über Veränderungen in der Beschaffenheit des dazwischen liegenden Gewebes abgeleitet werden können. Aus der Signalamplitude des Echos lässt sich die Gewebedämpfung bestimmen, woraus ebenfalls Informationen über die Beschaffenheit des Gewebes gewonnen werden können.

Über die Dopplerfrequenz der empfangenen Echos kann die Geschwindigkeit des Reflektors in Betrag und Richtung relativ zum Schallwandler bestimmt werden. Ist der Reflektor mit dem zu untersuchenden Organabschnitt fest verbunden, erhält man somit die Geschwindigkeit des sich bewegenden Organabschnitts und damit Informationen, die dem Tissue-Doppler entsprechen.

Mit der erfindungsgemäßen Anordnung ist es möglich, mit den begrenzten Ressourcen eines IMD eine Reihe von Parametern zu bestimmen, die in der Echographie oder bei Tissue-Doppler Untersuchungen bekannt und bewährt sind. Das IMD kann dabei als Implantat mit einer Therapiefunktion oder als ein reines Monitoring-Implantat zur Überwachung des Gesundheitszustands des Patienten ausgeführt sein.

Als Schallwandler können beispielsweise Piezokeramiken, piezoelektrische Polymerfolien oder kapazitive mikromechanische Wandler (CMUT) verwendet werden.

Die Ultraschallmessung kann sowohl kontinuierlich, in einem geeigneten Zeitraster oder intermittierend zu definierten Zeiten oder in definierten Zeitabständen erfolgen. Dabei können die Messungen auch mit Sensoren für andere physiologische Parameter oder mit physiologischen Ereignissen synchronisiert oder durch diese getriggert werden. Aus den mit der Ultraschallmessung gewonnenen Daten können geeignete Parameter bestimmt werden. Die Ermittlung der Parameter kann dabei im IMD selbst erfolgen oder in einem externen Gerät, das über eine Telemetrieverbindung in geeigneter Art und Weise mit dem IMD verbunden ist. Das IMD kann auch die ermittelten Parameter über die geeignete Telemetrieverbindung an das externe Gerät übertragen.

Die gewonnenen Daten oder die aus ihnen ermittelten Parameter können zu reinen Diagnosezwecken dienen oder zur Optimierung der Therapieparameter des IMD verwendet werden, wobei die Optimierung der Therapieparameter auch in einem geschlossen Regelkreis (Closed Loop) erfolgen kann. Die Optimierung der Therapieparameter kann direkt im IMD oder unter Einbeziehung des über eine Telemetrie mit dem IMD verbundenen externen Gerätes erfolgen. Darüber hinaus können die gewonnenen Daten, die daraus bestimmtem Parameter oder beides über einen geeigneten Zeitraum im IMD oder dem externen Gerät gespeichert werden, um sie zu einem geeigneten Zeitpunkt zur Verfügung zu stellen. Auch können aus den gewonnenen Daten, den Parametern oder einer Kombination aus beiden Trends oder Trendparameter ermittelt werden, die mit festgelegten Schwellwerten verglichen werden können, um beispielsweise Alarmmeldungen zu generieren. Die Ermittlung der Trends, der Vergleich mit den Schwellwerten und die Generierung der Alarme können vollständig oder teilweise im IMD oder vollständig oder teilweise im externen Gerät erfolgen.

Vorteile und Zweckmäßigkeiten der Erfindung ergeben sich im Übrigen aus der nachfolgenden Beschreibung von Ausführungsbeispielen anhand der Figuren. Von diesen zeigen:

Fig. 1A und 1B grafische Darstellungen von in einem Körper eines Patienten gewonnenen Ultraschallsignalen, wobei Fig. 1A ein typisches, störungsbehaftetes Ultraschall-Echosignal und Fig. 1B ein unter Einsatz eines erfindungsgemäßen Reflektors gewonnenes Ultraschall-Echosignal zeigt,
Fig. 2 eine Gesamtansicht einer ersten Ausführung einer erfindungsgemäßen Anordnung,
Fig. 3 eine Gesamtansicht einer zweiten Ausführung einer erfindungsgemäßen Anordnung und
Fig. 4 ein Blockschaltbild zur Erläuterung der Signalverarbeitung in einer weiteren Ausführungsform der Erfindung.

Fig. 1A und 1B verdeutlichen, welche Verbesserung der Signalqualität durch den Einsatz einer erfindungsgemäßen Vorrichtung, verglichen mit dem Stand der Technik, möglich ist.

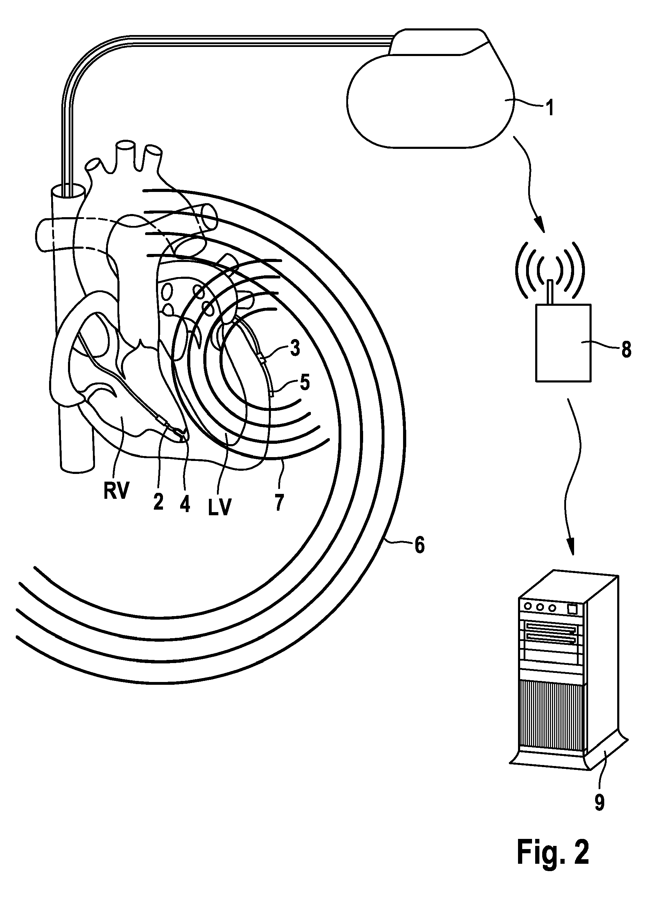

Fig. 2 zeigt ein IMD 1, das als Herzschrittmacher oder ICD ausgebildet ist, mit zwei Elektroden 2, 3, wobei die eine im rechten Ventrikel RV (RV-Elektrode) und die zweite im Coronarsinus am linken Ventrikel LV (LV-Elektrode) platziert ist. Die RV-Elektrode 2 enthält zusätzlich einen Schallwandler 4 zum Abstrahlen und Empfangen von Ultraschallimpulsen und die dafür notwendigen elektrischen Zuleitungen und Verbindungen (hier nicht separat dargestellt). Der Schallwandler weist dabei eine breite Richtcharakteristik auf, so dass an die Ausrichtung zum Reflektor nur geringe Anforderungen gestellt werden. Die LV-Elektrode 3 enthält zusätzlich an ihrem distalen Ende geeignete Ultraschallmarkierungen 5, die als Reflektor benutzt werden. An Stelle der LV-Elektrode mit Ultraschallmarkierung kann auch ein Stent mit Ultraschallmarkierung im Coronarsinus oder einer anderen geeigneten Position am LV platziert werden. Das IMD beinhaltet neben der üblichen funktionswesentlichen Elektronik zusätzlich Einheiten zur Erzeugung und zum Empfang von Ultraschallimpulsen und zur Auswertung der empfangenen Echos.

Das IMD 1 sendet über den Schallwandler 4 in der RV-Elektrode 2 einen Ultraschallimpuls 6 mit einer Frequenz von beispielsweise 1...5 MHz und einer Dauer von beispielsweise 1...10 $\mu$s aus, der sich mit der Schallgeschwindigkeit im Gewebe ausbreitet. Dieser Schallimpuls wird an Gewebestrukturen innerhalb und in der Umgebung des Herzens und an den Ultraschallmarkierungen 5 der LV-Elektrode 3 zurückgestreut, die als Ultraschallreflektoren wirken.

Die von den Ultraschallmarkierungen zurückgestreute Energie ist um ein vielfaches größer als die der Gewebestrukturen, so dass sich das Echo der Ultraschallmarkierungen 7 im Empfangssignal deutlich hervorhebt und beispielsweise durch einen einfachen Vergleich mit einem Amplitudenschwellwert von den Störsignalen getrennt werden kann, vgl. dazu Fig. 1B. Zusätzlich können Störsignale durch die Anwendung eines geeigneten Zeitfensters ausgeblendet werden, indem nur die Echos innerhalb dieses Zeitintervalls (beispielsweise nur zwischen 30 und 150 $\mu$s nach dem Aussenden des Schallimpulses) berücksichtigt werden. Die Schalllaufzeit wird über die Zeit zwischen dem Aussenden des Schallimpulses und dem Empfang des Echos der Ultraschallmarkierung mit allgemein bekannten Methoden bestimmt. Aus dieser Schallaufzeit wird die Entfernung zwischen dem Schallwandler und der Ultraschallmarkierung bestimmt.

Da sich sowohl Schallwandler als auch Ultraschallmarkierung an genau bekannten Orten im bzw. am Herzen befinden, kann aus der Entfernung zwischen beiden ein Maß für die Ausdehnung des LV gewonnen werden. Führt man die geschilderte Abstandsmessung z.B. mit einer Rate von 100 Messungen je Sekunde durch, erhält man die Größenänderung des LV über den Herzzyklus. Aus der Größenänderung des LV können geeignete Parameter, wie beispielsweise der enddiastolische und der endsystolische Abstand und daraus das Schlagvolumen, ermittelt werden.

Die Bestimmung der Parameter kann beispielsweise direkt im IMD erfolgen. Damit kann das IMD geeignete Therapieparameter, beispielsweise die AV- oder VV-Verzögerungszeit oder die Effektivität der Stimulation, automatisch anpassen. Um Energie zu sparen, kann diese Messung beispielsweise nur einmal stündlich für 20 Sekunden erfolgen. Die Abstandswerte oder die daraus ermittelten Parameter können im IMD gespeichert und über eine Telemetrieverbindung an ein Patientengerät 8 und ein Home-Monitoring Service-Center 9 übertragen werden. Aus den Parametern können beispielsweise Trendparameter bestimmt werden, die in ein Frühwarnsystem für die Verschlechterung der Herzinsuffizienz eingebunden werden können. Auch können die Parameter oder die Trendparameter mit einem Schwellwert verglichen werden, um bei einer kritischen Verschlechterung des Gesundheitszustandes eine Alarmmeldung für den Patienten oder den behandelnden Arzt zu erzeugen.

Eine weitere Ausführung beinhaltet ein IMD 1 und eine Elektrodenanordnung 2, 3 wie in Fig. 2. Anders als oben beschrieben, wird aber nicht die Laufzeit der Echos, sondern ihre Frequenzverschiebung durch den Doppler-Effekt ausgewertet. Bewegt sich die Ultraschallmarkierung 5 an der LV-Elektrode relativ zum Schallwandler 4, weisen die von ihr zurückgestreuten Echos eine Frequenzverschiebung gegenüber der Frequenz des gesendeten Ultraschallimpulses auf, die proportional zur Relativgeschwindigkeit zwischen Schallwandler und Ultraschallmarkierung ist (Doppler-Effekt).

Das Echo der Ultraschallmarkierung kann wiederum über eine einfache Amplitudenbewertung von den Störsignalen getrennt werden. Auch die Anwendung eines Zeitfensters ist wiederum möglich. Die Frequenzverschiebung (Doppler-Frequenz) wird dann vom IMD nach allgemein bekannten Verfahren aus dem Echo der Ultraschallmarkierung ermittelt. Da die Ultraschallmarkierung 5 über die Fixierung der LV-Elektrode mit der Wand des LV verbunden ist, kann aus der Dopplerfrequenz die Myokardgeschwindigkeit der Wand des LV relativ zum Schallwandler bestimmt werden, ähnlich wie bei der allgemein bekannten Tissue-Doppler Messung. Wird diese Geschwindigkeitsmessung beispielsweise mit einer Rate von 100 Messungen je Sekunde durchgeführt, erhält man den Verlauf der Myokardgeschwindigkeit des LV während des Herzzyklus. Aus diesem Geschwindigkeitsverlauf können Parameter bestimmt werden, die die Kinetik der Herzkontraktion beschreiben. Die Bestimmung der Parameter kann dabei im IMD oder in einem externen Gerät erfolgen.

Als ein solcher Parameter kann beispielsweise die aus der Tissue-Doppler Diagnostik bekannte maximale Myokardgeschwindigkeit während der Systole bestimmt werden. Anhand dieser Parameter können die Therapieparameter des IMD optimiert oder abgeglichen werden. Auch zeitliche Zusammenhänge zwischen dem intrakardialen Elektrogramm (IEGM) bzw. einem elektrischen Stimulus und der Myokardbewegung können als Parameter bestimmt werden. So kann beispielsweise aus dem Zeitabstand zwischen dem elektrischen Stimulus an der LV-Elektrode und dem Maximum der Myokardgeschwindigkeit des LV während der Systole die Reaktionszeit des LV ermittelt werden. Damit ist es beispielsweise möglich, die Ventrikel-zu-Ventrikel Verzögerung bei der Kardialen Resynchronisationstherapie (CRT) automatisch durch das IMD zu optimieren und anzupassen.

Die Geschwindigkeitsverläufe oder die daraus ermittelten Parameter können wiederum im IMD 1 in geeigneter Weise gespeichert und über eine Telemetrieverbindung an das externe Patientengerät 8 und ein Home-Monitoring Service-Center 9 übertragen werden. Aus den Parametern können Trendparameter bestimmt werden, die beispielsweise für das Monitoring des Krankheitsverlaufs verwendet oder in ein Frühwarnsystem eingebunden werden können. Auch können die Parameter oder die Trendparameter mit einem Schwellwert verglichen werden, um bei einer kritischen Verschlechterung des Gesundheitszustandes eine Alarmmeldung für den Patienten oder den behandelnden Arzt zu erzeugen.

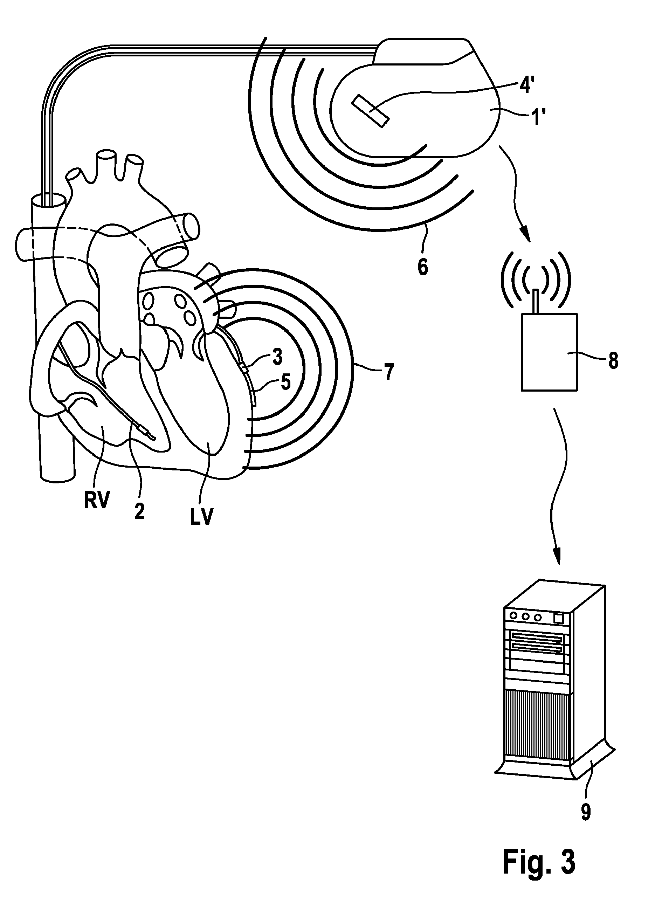

Eine weitere, in Fig. 3 gezeigte Ausführung beinhaltet ein IMD 1', das wiederum als Herzschrittmacher ausgebildet ist und einer der Anordnung nach Fig. 2 entsprechende Elektrodenanordnung mit einer RV-Elektrode 2 und einer LV-Elektrode 3 aufweist, die im Coronarsinus am LV (LV-Elektrode) platziert und mit einer Ultraschallmarkierung 5 versehen ist. An Stelle der LV-Elektrode mit Ultraschallmarkierung kann auch ein anderes Implantat mit einer Ultraschallmarkierung (beispielsweise ein Stent) im Coronarsinus an geeigneter Position platziert werden. Das IMD enthält zusätzlich mindestens einen Schallwandler 4' zur Abstrahlung und zum Empfang von Ultraschallwellen vorzugsweise in die Richtung der Ultraschallmarkierung am LV. Diese Anordnung hat den Vorteil, dass keine spezielle Elektrodenleitung, die einen Schallwandler und spezielle Zuleitungen enthält, erforderlich ist.

Das IMD 1 sendet über den enthaltenen Schallwandler 4' einen Schallimpuls 6 aus, der sich im Gewebe ausbreitet. Aufgrund der größeren Entfernung zwischen Schallwandler und Reflektor ist hier eine niedrigere Schallfrequenz, beispielsweise im Bereich zwischen 100 kHz und 1 MHz, wegen der hierbei geringeren Dämpfung im Gewebe günstiger. Dementsprechend ist eine vergrößerte Impulslänge von 10...100 $\mu$s empfohlen. Dieser Schallimpuls wird an Gewebestrukturen und an der Ultraschallmarkierung 5 im CS zurückgestreut.

Die von der Ultraschallmarkierung zurückgeworfene Energie ist wiederum größer als die an den Gewebestrukturen zurückgestreute, so dass sich das Echo der Ultraschallmarkierung 7 im Empfangssignal deutlich hervorhebt und beispielsweise mit einem einfachen Amplitudenschwellwert von den Störsignalen getrennt werden kann. Zusätzlich können Störsignale durch die Anwendung eines geeigneten Zeitfensters ausgeblendet werden, indem nur die Echos innerhalb dieses Zeitintervalls (beispielsweise nur zwischen 100 und 300 $\mu$s nach dem Aussenden des Schallimpulses) berücksichtigt werden. Als weiteres Kriterium für die Trennung der Echos der Ultraschallmarkierung von den Störsignalen kann die Dopplerverschiebung der Echos verwendet werden. Die Echos der mit dem schlagenden Herzen verbundenen Ultraschallmarkierungen weisen die größten Dopplerverschiebungen auf, da die Herzwände die sich am schnellsten bewegende Struktur im Brustkorb darstellen. Alle anderen Strukturen, die ein Echosignal erzeugen, wie beispielsweise die Lungenbläschen oder die Hautoberfläche, bewegen sich viel langsamer und weisen deshalb eine viel geringere Dopplerverschiebung auf und können mit allgemein bekannten Methoden, beispielsweise durch Filterung, von den Echos der Ultraschallmarkierung getrennt werden.

Die Schalllaufzeit wird über die Zeit zwischen dem Aussenden des Schallimpulses und dem Empfang des Echos der Ultraschallmarkierung mit allgemein bekannten Methoden bestimmt. Aus dieser Schalllaufzeit wird die Entfernung zwischen dem Schallwandler im IMD und der Ultraschallmarkierung bestimmt. Führt man die geschilderte Abstandsmessung z.B. mit einer Rate von 100 Messungen je Sekunde durch, erhält man Informationen über die Bewegung des LV während des Herzzyklus. Möglich ist auch die Bestimmung der Dopplerverschiebung der Echos der Ultraschallmarkierung, aus der Informationen über die Geschwindigkeit des LV und die Kontraktionsdynamik des LV gewonnen werden können. Aus den Abstands- oder Geschwindigkeitsverläufen können wiederum geeignete Parameter im IMD oder in einem externen Gerät bestimmt werden.

Die Verläufe oder die daraus bestimmten Parameter können für die Optimierung der Therapieparameter des IMD verwendet werden, im IMD gespeichert werden, über eine Telemetrieverbindung an ein externes Patientengerät 8 und ein Service Center 9 übertragen werden und es können daraus geeignete Trendparameter ermittelt werden, die beispielsweise für das Monitoring des Krankheitsverlaufs verwendet oder in ein Frühwarnsystem eingebunden werden können. Auch können die Parameter oder die Trendparameter mit einem Schwellwert verglichen werden, um bei einer kritischen Verschlechterung des Gesundheitszustandes eine Alarmmeldung für den Patienten oder den behandelnden Arzt zu erzeugen.

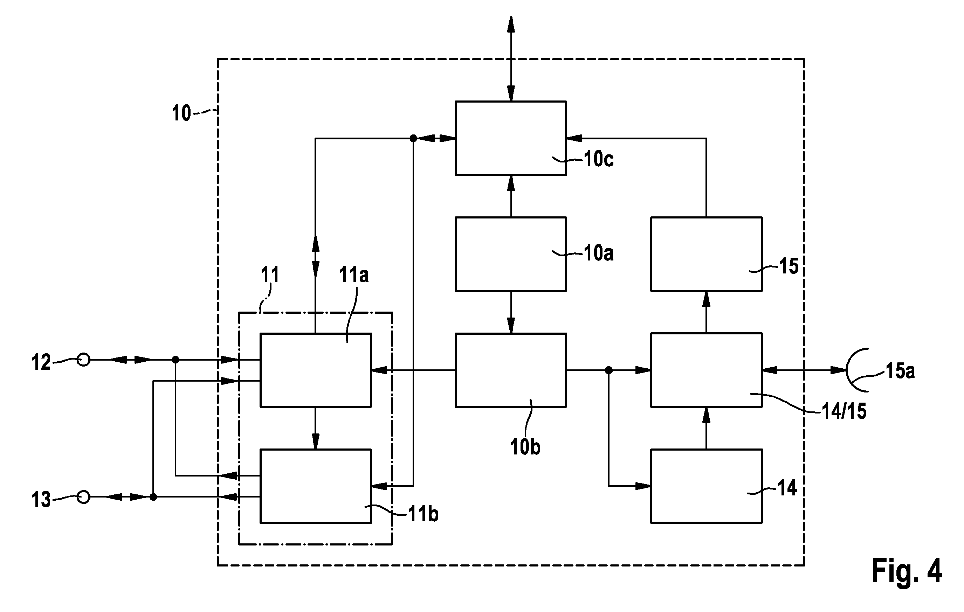

Fig. 4 zeigt schematisch in Art eines Blockschaltbildes wesentliche Funktionselemente eines implantierbaren elektromedizinischen Gerätes 10 gemäß einer Ausführung der Erfindung, welches etwa in eine Gesamtanordnung der in Fig. 3 gezeigten Art eingebunden sein kann. Das Gerät 10 umfasst eine Schrittmacherkomponente 11, die mit Sensing- und Stimulationselektroden 12, 13 verbunden ist, eine Ultraschallgeneratorkomponente 14 und eine Akustikmesskomponente 15.

Die Schrittmacherkomponente 11 umfasst einen Sensing-Teil 11a und einen Stimulations-Teil 11 b, welche in der dargestellten Ausführung beide jeweils mit den Elektroden 12 (RV-Elektrode) und 13 (LV-Elektrode) verbunden sind, um Herzaktionspotentiale im jeweiligen Ventrikel zu erfassen und erforderlichenfalls an diesen Stimulationsimpulse abzugeben. Es versteht sich, dass der Sensing-Teil 11a in üblicher Weise ausgangsseitig mit einem Steuereingang des Stimulations-Teils 11 b verbunden ist.

Ein Controller 10a steuert die aufeinander abzustimmenden Funktionen des Gerätes 10 insgesamt und speziell über eine Synchronisationsstufe 10b die zeitliche Synchronisation zwischen Detektionsvorgängen des Sensing-Teils 11a des Herzschrittmachers 11 und der Akustikmesskomponente 15, die über eine Umschaltstufe 14/15 eingangsseitig mit einem Schallwandler 15a verbunden werden kann, der etwa eine Piezokeramik oder eine piezoelektrische Polymerfolie aufweist. Auch der Betrieb des ebenfalls über die Umschaltstufe 14/15 mit dem Schallwandler 15a verbindbaren Ultraschallgenerators 14 wird durch die Synchronisationsstufe 10b getriggert.

Der Controller 10a steuert schließlich auch den Betrieb einer Telemetriestufe 10c, über die sowohl der Sensing-Teil 11a als auch der Stimulations-Teil 11b des Herzschrittmachers und die Akustikmesskomponente 15 mit einem externen Patientengerät verbunden sind, um Messergebnisse von Herzaktionspotentialen sowie der akustischen Messungen dorthin zu liefern und Steuerbefehle für den Herzschrittmacher von dort zu erhalten. In diesen Steuerbefehlen können sich Ergebnisse einer externen Auswertung der Messungen der Akustikmesskomponente 15 widerspiegeln, für die bei der hier gezeigten Ausführung keine interne Rückkopplung in den Schrittmacherteil vorgesehen ist. Alternativ hierzu kann aber auch eine geräteinterne Auswertung der akustischen Messungen, mit direkter Beeinflussung des Schrittmacherbetriebes durch deren Ergebnisse, vorgesehen sein. Mindestens eine der Elektroden 12, 13 wirkt dann als Aktor einer Schrittmachertherapie.

Auch im Übrigen ist die Erfindung nicht auf die hier gezeigten Beispiele und hervorgehobenen Aspekte beschränkt, sondern ebenso in einer Vielzahl von Abwandlungen ausführbar, die im Rahmen fachgemäßen Handels liegen.

**Patentansprüche**

1. Implantierbare Messanordnung zur intrakorporalen akustischen Messung von geometrischen Parametern und/oder Bewegungsparametern in und an Organen und/oder Geweben eines Patienten, aufweisend:

    ein implantierbares elektromedizinisches Gerät;

    einen oder mehrere implantierbare Schallwandler zum Abstrahlen und Empfangen von Ultraschallwellen, die in Signalverbindung mit dem implantierbaren Gerät stehen; und

    einen vom Schallwandler entfernt anzuordnenden implantierbaren Reflektor zum Zurückstrahlen der Ultraschallwellen in die Richtung des Schallwandlers,

    wobei das elektromedizinische Gerät Mittel zur Auswertung der vom Schallwandler aufgenommenen zurückgestrahlten Ultraschallwellen aufweist.

2. Messanordnung nach Anspruch 1 , wobei der Reflektor als einem Organ bzw. Gewebe des Patienten im implantierten Zustand räumlich fest zuordenbares, mindestens teilweise hochgradig ultraschallreflektierendes, Implantat ausgebildet ist.

3. Messanordnung nach Anspruch 1 , wobei der Schallwandler einen Ultraschallimpulsgenerator aufweist und die erzeugten Ultraschallimpulse eine Frequenz zwischen 0,1 und 5 MHz haben.

4. Messanordnung nach einem der vorhergehenden Ansprüche, wobei der Schallwandler Mittel zur Umschaltung vom Sende- in den Empfangsbetrieb aufweist.

5. Messanordnung nach einem der vorhergehenden Ansprüche, wobei der Schallwandler eine Piezokeramik oder eine piezoelektrische Polymerfolie aufweist oder als kapazitiver mikromechanischer Wandler (CMUT) ausgeführt ist.

6. Messanordnung nach einem der vorhergehenden Ansprüche, mit einem zusätzlichen Sensor zum Detektieren von Gewebe- und/oder Organmesssignalen, welcher mit dem implantierbaren Gerät verbunden und an einem Gewebe und/oder Organ des Patienten angeordnet ist, wobei das implantierbare Gerät Mittel zur Synchronisierung der akustischen Messung mit dem Betrieb des Sensors aufweist.

7. Messanordnung nach Anspruch 6, wobei das implantierbare Gerät zusätzlich Mittel zum Triggern der akustischen Messung durch den zusätzlichen Sensor aufweist.

8. Messanordnung nach einem der vorhergehenden Ansprüche, mit einem Aktor zur Anwendung einer Therapie im Ansprechen auf Ergebnisse der akustischen Messung aufweist.

9. Anordnung nach einem der vorhergehenden Ansprüche, wobei der Schallwandler zur Fixierung an dem implantierbaren Gerät und der Reflektor zur Fixierung am Gewebe und/oder am Organ des Patienten, ausgebildet sind.

10. Messanordnung nach einem der Ansprüche 1 bis 9, wobei das implantierbare Gerät ein Herzschrittmacher ist, der Schallwandler am distalen Ende einer Elektrodenleitung, die in einem Ventrikel ausgebildet ist, und der Reflektor am distalen Ende einer Elektrodenleitung, die im oder an dem anderen Ventrikel platziert ist, ausgebildet ist, derart, dass die Anordnung zur intrakardialen Messung der Größenänderung der Ventrikel über den Herzzyklus und/oder zur intrakardialen Messung der Myokardgeschwindigkeit ausgebildet ist.

11. Messanordnung zur Fernüberwachung von physiologischen Messgrößen eines Patienten, aufweisend:

    eine implantierbare Messanordnung zur intrakorporalen akustischen Messung von geometrischen Parametern und Bewegungsparametern in und/oder an Organen und/oder Geweben des Patienten nach einem der Ansprüche 1 bis 11;
    eine mit der implantierbaren Anordnung gekoppelte externe Übertragungseinheit zum Empfangen, Speichern und zur Weiter-Übertragung der Messsignale und Steuersignale, die in Telemetrieverbindung mit der implantierbaren Anordnung steht; und
    eine mit der externen Übertragungseinheit verbundene Fernverarbeitungseinheit zum Empfangen der Messsignale aus der externen Übertragungseinheit, zur Verarbeitung der empfangenen Messsignale und zum Steuern die implantierbare Anordnung im Ansprechen auf Ergebnisse der Verarbeitung der Messsignale durch Steuersignale.

Fig. 1a

Fig. 1b

**Fig. 2**

Fig. 4

Europäisches
Patentamt

European
Patent Office

Office européen
des brevets

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2005/049492 A1 (SWEENEY ROBERT J [US] ET AL) 3. März 2005 (2005-03-03) * Absätze [0012], [0013], [0015], [0016], [0024]; Abbildung 1 * ----- | 1,8,9,11 | INV. A61B8/08 |
| Y,D | US 6 421 565 B1 (HEMMINGSSON TRYGGVE [SE]) 16. Juli 2002 (2002-07-16) * Anspruch 1 * * Spalte 5, Zeilen 1-13,47-49,61-63 * * Spalte 7, Zeilen 26-30 * * Abbildung 1 * ----- | 1-7,10 | |
| Y | US 2007/167758 A1 (COSTELLO BENEDICT J [US]) 19. Juli 2007 (2007-07-19) * Absätze [0040] - [0044], [0052] * * Abbildungen 1,3,5,6 * ----- | 1-7,10 | |
| A,D | US 6 795 732 B2 (STADLER ROBERT W [US] ET AL) 21. September 2004 (2004-09-21) * Spalte 15, Zeilen 58-60; Abbildungen 1,2 * ----- | 1,3,11 | |
| A | US 5 840 028 A (CHUBACHI NORIYOSHI [JP] ET AL) 24. November 1998 (1998-11-24) * Spalte 2, Zeile 55 - Spalte 3, Zeile 17; Abbildung 1 * ----- | 1 | RECHERCHIERTE SACHGEBIETE (IPC) A61B A61N |
| A | US 2007/167739 A1 (SALO RODNEY W [US]) 19. Juli 2007 (2007-07-19) * Absätze [0002] - [0004]; Abbildungen 2,3 * ----- | 1,10 | |
| A | WO 2008/050334 A (CARDIOGAL LTD [IL]; SHARF YEHUDA [IL]; LANCASTER GILEAD I [US]) 2. Mai 2008 (2008-05-02) * Seite 19, Zeilen 14-23 * * Seite 29, Zeilen 7-11 * * Seite 34, Zeilen 13-19 * * Abbildungen 5a-5c * ----- | 1 | |

Der vorliegende Recherchenbericht wurde für alle Patentansprüche erstellt

| Recherchenort | Abschlußdatum der Recherche | Prüfer |
|---|---|---|
| Den Haag | 14. April 2009 | Mecking, Nikolai |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 09 15 1323

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-04-2009

| Im Recherchenbericht angeführtes Patentdokument | Datum der Veröffentlichung | Mitglied(er) der Patentfamilie | Datum der Veröffentlichung |
|---|---|---|---|
| US 2005049492    A1 | 03-03-2005 | KEINE | |
| US 6421565    B1 | 16-07-2002 | DE     69825284 D1<br>DE     69825284 T2<br>EP     0999789 A1<br>WO     9907285 A1 | 02-09-2004<br>21-07-2005<br>17-05-2000<br>18-02-1999 |
| US 2007167758    A1 | 19-07-2007 | KEINE | |
| US 6795732    B2 | 21-09-2004 | US     2003083702 A1<br>WO     03037177 A2<br>US     2004176810 A1 | 01-05-2003<br>08-05-2003<br>09-09-2004 |
| US 5840028    A | 24-11-1998 | JP     3652791 B2<br>JP     10005226 A | 25-05-2005<br>13-01-1998 |
| US 2007167739    A1 | 19-07-2007 | KEINE | |
| WO 2008050334    A | 02-05-2008 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5544656 A
- US 6421565 B
- US 6795732 B
- US 2005027323 A
- US 4805628 A
- US 5383466 A
- US 5921933 A
- US 6506156 B
- US 7014610 B
- US 5289831 A
- WO 2004103207 A